# EUROPEAN PATENT APPLICATION

(11) **EP 1 693 082 A1**
(43) Date of publication of application: **23.08.2006**
(21) Application number: 04807219.3
(22) Date of filing: 10.12.2004
(51) Int. Cl.: A61M 25/10

(54) **BALLOON CATHETER**

(30) Priority: 11.12.2003 JP 2003413283
(71) Applicant: IR Medical Laboratory Co., Ltd., Koriyama-shi, Fukushima 963-8871 (JP); Hangai, Masanori, Kyoto-shi, Kyoto 6028014 (JP)
(72) Inventor: HANGAI,M, Urban life 104 159,Kageyukouji-cho, Kamigyo-ku, Kyoto-shi, Kyoto (JP); KAWABE, Daisuke, Koriyama-shi, Fukushima 963-8871 (JP)
(74) Representative: Godwin, Edgar James
(86) International application number: PCT/JP2004/018861
(87) International publication number: WO 2005/056101

(57) **Abstract**

A balloon catheter for medical treatments easily manufacturable at low cost by a rather simple structure by providing a smaller diameter non-profile balloon catheter by eliminating the need for adhesion and welding of a balloon and eliminating extra profile at a connection part between the balloon and a shaft, wherein PTFE may be used for the shaft, and silicone may be used for the balloon. An inside hollow shaft (3) formed of a balloon material is inserted into an outside hollow shaft (2) formed of a shaft material, and the tip part of the inside hollow shaft (3) is projected from the tip end of the outside hollow shaft (2) to manufacture a double-tube catheter shaft (2). An expandable balloon (5) is formed of the projected portion of the inside hollow shaft (3), a cap (4) is fitted to the tip of the inside hollow shaft (3), and the PTFE is used for the outside hollow shaft (2), with the silicone used for the inside hollow shaft (3).

## Description

### TECHNICAL FIELD

This invention relates to a balloon catheter for medical treatments, and more particularly, to a balloon catheter effectively applicable to intra-vascular treatments.

### BACKGROUND ART

A balloon catheter is used for the following intra-vascular treatments, for instance. That is, (1) treatments (PTA) adaptable to maintain circulation of the blood by effecting transcutaneous insertion of the balloon catheter into blood vessels for dilatation to vascular strictures, (2) treatments adaptable to catheterize stents for the purpose of providing vascular dilatation, (3) treatments adaptable to remove voluminously-attached thrombus from the blood vessels, (4) intra-vascular treatments in which the balloon catheter temporarily serves as a cover of an embolus (such as coil) at the time when aneurysm etc. is filled with the embolus, (5) treatments adaptable to provide reflux of water gathered in a cerebral ventriculus by drilling an opening in a ventriculus tertius through the medium of an endoscope in hydrocephalic cases etc., and other treatments.

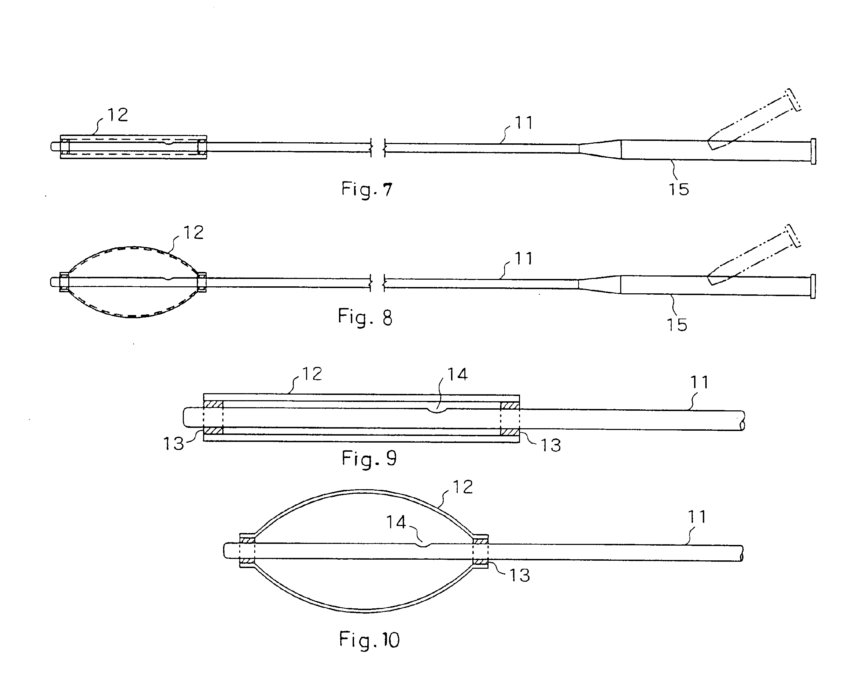

FIGS. 7 to 10 illustrate one conventional balloon catheter, which has a cylindrical balloon 12 at the outside of the tip part of a long shaft 11 having a circular form in section. The opposite ends of the balloon 12 are secured to an outside face of the shaft 11 with an adhesive 13 or by welding, or alternatively, are tied thereto with a string etc. The shaft 11 has, at its outside face portion fitted with the balloon 12, an air or like fluid supply and exhaust opening 14, so that expandable actions of the balloon 12 may be caused by supply and exhaust of air or like fluid to and from a space between the shaft 11 and the balloon 12.

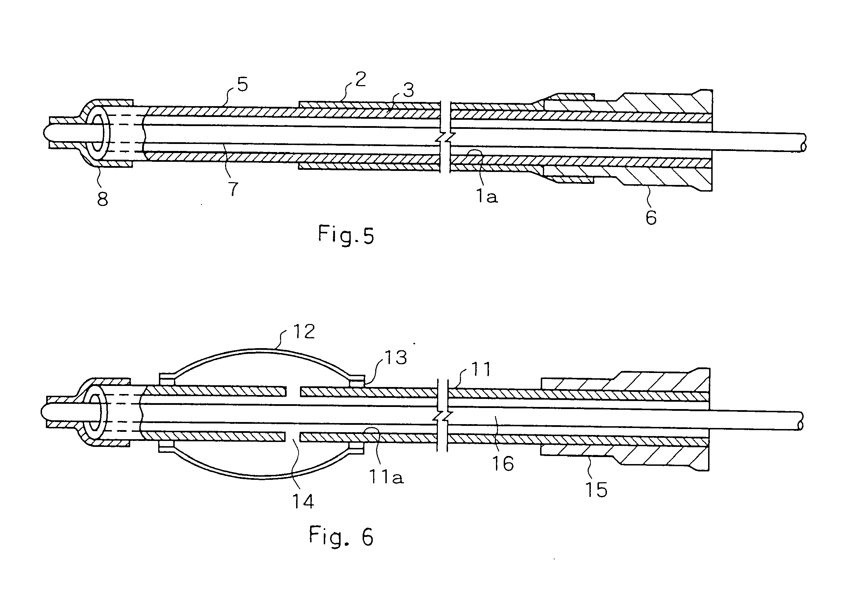

A shaft having a single lumen, for instance, is used for the shaft 11. A connector 15 formed of a metal- or synthetic resin-made hard tube is connected to the basal end of the shaft 11, permitting the air or like fluid for balloon expandable actions to be supplied to the supply and exhaust opening 14 through the intra-shaft lumen. Further, as one of applications of the conventional balloon catheter, there is one instance of use as a balloon catheter structured to be integral with a guide wire by inserting a guide wire 16 into a lumen 11a, as shown in FIG. 6.

In the above types of conventional balloon catheters, a flexible material such as nylon and polyurethane is used for the shaft 11. Coating is sometimes given to a shaft surface in order to provide more smoothed sliding. By the way, a flexible and expansible material such as nylon, polyethylene-polyolefin copolymer, polyethylene-terephthalate and natural rubber is used for the balloon 12.

Meanwhile, a prior art reference relating to the present invention includes the following patent documents 1 to 4. The invention according to the patent document 1 relates to an anti-thrombotic balloon catheter, specifically, a balloon catheter structured so that a catheter tube has, at its tip part, a balloon mounting portion of a diameter smaller than the outer diameter of a main body, wherein, with a balloon mounted to the balloon mounting portion, the opposite ends of the balloon are secured to the balloon mounting portion with an adhesive etc., and clearances between the balloon and the catheter tube at the opposite ends of the balloon mounting portion are sealed up by filling the above clearances with an anti-thrombotic elastomer of a type being hardened at room temperature.

The invention according to the patent document 2 relates to a method of manufacturing a balloon catheter involving use of silicone rubber, specifically, a method, in which a first tube having a lumen for urination etc. and a lumen for a fluid for balloon expandable actions is formed by primary extrusion molding, and a mold release agent is applied to a first tube portion adapted to formation of a balloon, before a second tube is formed on the first tube by secondary extrusion molding, permitting the tip part of the second tube to function as the balloon in such a manner as to expand the tip part of the second tube by supply of the fluid for balloon expandable actions to the resultant tube generally having the same outer diameter.

The invention according to the patent document 3 relates to a seal structure and a sealing method of an expandable balloon catheter particularly suited to treatments for intracerebral small-diameter blood vessels, specifically, a seal structure and a sealing method both applicable to a case where a balloon formed of a flexible material such as silicone having difficulty in being secured by adhesion and welding needs to be secured to a catheter body formed of a typical catheter material such as polyurethane in such a manner that, with an inside tubular member arranged in a coaxial position at the inside of an outside tubular member, the tip part of the inside tubular member is projected from the tip end of the outside tubular member to place the balloon at a projected portion, and the opposite ends of the balloon are respectively secured to the tip part of the outside tubular member and the tip part of the inside tubular member with an adhesive layer, a holding ring and a sleeve.

The invention according to the patent document 4 relates to a temporarily intra-vascular blocking balloon catheter that may function as a guide wire to ensure excellent operability adaptable to treatments for complicatedly branching blood vessels such as coronary arteries and cerebral blood vessels, and is also capable of being inserted into the blood vessels in such a manner as to follow the guide wire, permitting sufficient insertion up to vascular peripheries, specifically, a balloon catheter having a balloon formed of a highly expansible material (such as thermoplastic polyurethane and silicone) providing 300 to 1100% in percentage elongation after fracture, and a shaft formed of a highly elastic material (such as stainless steel) providing 1GPa or above in modulus of bending elasticity with an outer diameter in the range of 0.3556 to 0.4572 mm, wherein the balloon catheter has, only at its tip part, a lumen adapted to follow the guide wire.
[Patent document 1] Japanese Patent Laid-open 2001-29453
[Patent document 2] Japanese Patent Laid-open 2002-210017
[Patent document 3] Japanese Patent Laid-open 2001-238957
[Patent document 4] Japanese Patent Laid-open 2003-250898

In the case of the conventional balloon catheter shown in FIGS. 7 to 10, a shaft thickness is increased in accordance with the thickness of the adhesive or the string etc. In particular, when tying is taken, a quite increase in shaft thickness is caused. Further, the adhesive is limited in type of available materials. The same may be said of the welding, that is, a difficulty arises in welding depending on the type of welding materials, leading to limitations in type of available materials. Furthermore, two lumens, one for the fluid for balloon expandable actions, and the other for instruments, are required for the shaft, leading to an increase in shaft thickness due to need for the above two lumens as well.

When the shaft whose surface having been given coating to provide more smoothed sliding is used, a long-term manual operation sometimes causes the coating to be worn off depending on the type of coating. In addition, coating with an animal coating agent (heparin) leads to need for strict management in considerations of the safety.

Further, use of a low compliant (or low expansible) material (such as polyethylene-terephthalate) as the balloon material needs to keep the balloon folded not to be spread out before its expansion in particular, resulting in an increase in profile thickness in accordance with the thickness of a folded-up balloon portion.

In the case of the inventions according to the above patent documents 1 to 4, a complicated structure is required for a portion adapted to secure the balloon, or time is taken for machining or manufacturing, resulting in an increase in cost.

### DISCLOSURE OF THE INVENTION

The present invention has been undertaken to solve the above problems, and is intended to, in a balloon catheter for medial treatments, a balloon catheter that is easily manufacturable by a rather simple structure for a reduction in cost by providing a smaller diameter non-profile balloon catheter by eliminating the need for adhesion and welding etc. of a balloon and eliminating extra profile at a connection part between the balloon and a shaft, and further, enables use of PTFE for the shaft and silicone for the balloon.

A balloon catheter according to Claim 1 of the present invention relates to a balloon catheter having an expandable balloon at the tip part of a shaft, and characterized in that a hollow shaft formed of a balloon material is inserted into a hollow shaft formed of a shaft material, and the tip part of the inside hollow shaft is projected from the tip end of the outside hollow shaft so that the expandable balloon is formed of a projected portion of the inside hollow shaft.

The outside and inside hollow shafts are in the form of outside and inside tubes requiring that the inside tube is longer than the outside tube by a length corresponding to a balloon length, in which case, a double-tube catheter shaft is manufactured by expansion molding, heat contraction and immersion etc., thereby providing the double-tube catheter shaft structured so that the outside and inside hollow shafts are integrally in close contact with each other. The tip part of the inside hollow shaft is projected from the outside hollow shaft by a prescribed length, so that the projected portion at the tip end of the inside hollow shaft may be expanded into the form of the balloon by supply of air or like fluid for balloon expandable actions into the inside hollow shaft through the shaft basal end. The tip end of the inside hollow shaft is closed up to prevent the supplied air etc. from leaking out. It is to be noted that the tip part of the inside hollow shaft is also adaptable to be housed in a folded form within the tip part of the outside hollow shaft. Further, it is allowable to use the balloon catheter for various purposes by inserting a guide wire and medical instruments etc. into the inside hollow shaft.

In the balloon catheter according to Claim 1, the balloon catheter according to Claim 2 of the present invention relates to a balloon catheter characterized in that the tip end of the inside hollow shaft is closed up with a cap formed of the shaft material. Specifically, while the tip end of the inside hollow shaft may be closed up by adhesion and welding etc. after being crushed flat, it is preferably necessary to close up the tip end of the inside hollow shaft in such a manner as to insert the tip end of the inside hollow shaft into a bottomed cap (formed of the same shaft material as the outside hollow shaft material, for instance) of a prescribed length.

In the balloon catheter according to Claim 1 or 2, the balloon catheter according to Claim 3 of the present invention relates to a balloon catheter characterized in that after insertion of a guide wire or other core material into the inside hollow shaft, the guide wire is secured to the tip part of the inside hollow shaft. It is preferably necessary to secure the tip part of the guide wire or other core material by taking advantage of the above cap, for instance, in which case, the tip end of the guide wire or other core material needs to be projected outwards. The balloon catheter according to Claim 3 is specified as one of applications of the balloon catheter of the present invention, in other words, one instance of a balloon catheter structured to be integral with the guide wire or other core material. This type of balloon catheter unites a balloon catheter function and a guide wire function.

In the balloon catheter according to Claim 3, the balloon catheter according to Claim 4 of the present invention relates to a balloon catheter characterized in that the guide wire or other core material is provided with one or more lumens. The lumen is specified as a lumen that passes through the guide wire or other core material. The balloon catheter according to Claim 4 is one instance where the balloon catheter of the present invention may be provided in the form of a balloon catheter having more than one lumen when the guide wire or other core material is used, permitting injection of a contrast medium and medicines etc. to a portion ahead of the catheter.

In the balloon catheter according to any one of Claims 1 to 3, the balloon catheter according to Claim 5 of the present invention relates to a balloon catheter characterized in that the inside hollow shaft is provided with more than one lumen. The balloon catheter in this case is also one instance where the balloon catheter of the present invention may be provided in the form of the balloon catheter having more than one lumen, permitting injection of the contrast medium and medicines etc. to a portion ahead of the catheter.

In the balloon catheter according to any one of Claims 1 to 5, the balloon catheter according to Claim 6 of the present invention relates to a balloon catheter characterized in that the outside hollow shaft is formed of polytetrafluoroethylene (which will be hereinafter simply referred to as PTFE). That is, while a conventionally-available flexible and non-expansible material such as nylon and polyurethane may be used for the outside hollow shaft, the balloon catheter of the present invention enables the PTFE having difficulty in being adapted to high-power adhesion and welding to be used for the outside hollow shaft, because of its structure eliminating the need for adhesion and welding of the balloon to the shaft.

In the balloon catheter according to any one of Claim 1 to 6, the balloon catheter according to Claim 7 of the present invention relates to a balloon catheter characterized in that the inside hollow shaft is formed of silicone. That is, while a conventionally-available flexible and expansible material such as nylon, polyethylene-polyolefin copolymer, polyethylene-terephtalate and natural rubber may be used for the balloon, the balloon catheter of the present invention enables the silicone having difficulty in being adapted to high-power adhesion and welding to be used for the balloon, because of its structure eliminating the need for adhesion and welding of the balloon to the shaft.

In the balloon catheter according to any one of Claims 1 to 5 and 7, the balloon catheter according to Claim 8 of the present invention relates to a balloon catheter characterized in that the outside hollow shaft is formed of soft metal. That is, the balloon catheter of the present invention enables the soft metal such as Ni-Ti shape memory alloys and super-elastic alloys to be used for the outside hollow shaft, permitting the balloon catheter itself to be used as the guide wire.

The balloon catheter of the present invention having the above structure requires that the inside hollow shaft formed of the balloon material passes through the whole of the outside hollow shaft, and that a portion other than a specific portion adapted to formation of the balloon is pressed into contact with the outside hollow shaft to form an integral unit, eliminating extra profile at a connection part between the balloon and the shaft, unlike the conventional technology, thus permitting a smaller diameter balloon catheter to be manufactured. Further, the balloon catheter of the present invention enables use of the shaft having the single lumen adaptable to supply of the fluid for balloon expansible actions into the inside hollow shaft and insertion of the instruments such as the guide wire, permitting a further reduction in outer diameter of the shaft. Furthermore, use of the smaller diameter balloon catheter for manual operations enables lower invasive treatments to be performed. Furthermore, the balloon catheter of the present invention only needs to provide the inside hollow shaft within the outside hollow shaft, permitting easy manufacturing by the rather simple structure, leading to a reduction in cost.

The balloon catheter of the present invention may be provided in the form of a non-profile balloon catheter structured to be integral with the guide wire or other core material by inserting the guide wire or other core material into the inside hollow shaft, or alternatively, may be given strength as much as the guide wire by using the soft metal etc. for the outside hollow shaft, permitting the non-profile balloon catheter itself to function also as the guide wire, leading to attainment of treatments etc. of high efficiency. Further, the non-profile balloon catheter may be used for injection of the contrast medium and medicines etc. to the portion ahead of the catheter by providing one or more lumens for the guide wire or other core material, or alternatively, by providing the inside hollow shaft in the form of a shaft having more than one lumen.

The balloon catheter of the present invention enables use of the PTFE as the material of the outside hollow shaft, in addition to the conventionally available materials. While the PTFE is excellent in durability and anti-thrombus, use of the PTFE for the catheter has been supposed to be difficult, because of its difficulty in being adapted to high-power adhesion and welding, like the silicone. In addition, the PTFE is available as one of fluorine-contained resins, so that smooth sliding may be obtained, leading to no need for coating adapted to provide the smooth sliding.

Further, the balloon catheter of the present invention enables use of the silicone as the material of the balloon, in addition to the conventionally available materials. The silicone is too difficult for the shaft member to be adapted to high-power adhesion and welding, while an attempt to use the silicone as the balloon results in limitations in balloon securing means to tying. Further, the silicone has the property of permitting air to pass through. Thanks to this silicone property, removal of air is facilitated, permitting the balloon catheter to be applied to areas inclusive of cerebral surgical areas having been supposed to be difficult to take advantage of the balloon catheter in serious considerations of the presence of air. Furthermore, the silicone may be machined into a product of quite small thickness (an already confirmed thickness at present =20 µm: Asahi Rubber Co. Ltd.). Thus, a reduction in inner diameter of the catheter by the presence of the silicone passing through the inside of the whole shaft leads to no serious obstacle. The silicone provides high compliance (expandability). Thus, there is no need to fold up the balloon before its expansion, permitting a further reduction in shaft thickness. Even if the need to fold up the balloon arises, an increase in profile thickness more than necessary does not occur because the balloon is adaptable to be housed within the shaft.

The balloon catheter of the present invention described the above produces the following effects.
(1) The balloon catheter of the present invention is of the structure requiring that the inside hollow shaft formed of the balloon material is inserted into the outside hollow shaft so that the balloon is formed of the tip part of the inside hollow shaft at the tip part of the outside hollow shaft, providing the non-profile balloon catheter that eliminates the need for adhesion and welding etc. of the balloon, unlike the conventional technology, then eliminating the extra profile at the connection part between the balloon and the shaft, thus permitting the balloon at the time when being in a non-expanded state to be located at the inside away from the outside face of the shaft.
(2) The shaft having the single lumen may be used in the non-profile balloon catheter, permitting a smaller diameter non-profile balloon catheter to be obtained.
(3) Use of the above structure makes it possible to manufacture a superfine balloon catheter having been supposed to be impossible of realization by the conventional structure, permitting treatments involving use of the balloon catheter to be performed in areas such as cerebral-surgical and ophthalmologic areas (fine vascular peripheries such as intracranial vascular peripheries and retinal vein centrales) where the conventionally available balloon catheter has failed to reach.
(4) Further, the balloon catheter of the present invention provides the advantage that use of the small diameter non-profile balloon catheter enables the lower invasive treatments than the existing conditions to be performed even in areas where the conventionally available balloon catheter has already succeeded in reaching.
(5) The balloon catheter of the present invention may be provided in the form of the non-profile balloon catheter structured to be integral with the guide wire or other core material, or alternatively, may be given strength as much as the guide wire by using the soft metal etc. for the outside hollow shaft, permitting the non-profile balloon catheter itself to function also as the guide wire, leading to attainment of treatments etc. of high efficiency.
(6) The non-profile balloon catheter may be used for injection of the contrast medium and medicines etc. to the portion ahead of the catheter by providing one or more lumens for the guide wire or other core material, or alternatively, providing the inside hollow shaft in the form of the shaft having more than one lumen.
(7) The PTFE having been not available up to now because of its difficulty in being adapted to adhesion and welding may be used as the shaft material. As compared with other plastic materials, the PTFE is remarkably excellent in vital adaptability (anti-thrombus), surface smoothness, heat resistance, chemical resistance, and durability etc., eliminating the need for coating adapted to provide the smooth sliding, thus permitting the shaft most suitable to the balloon catheter for medical treatments to be obtained.
(8) The silicone having been not available up to now because of its difficulty in being adapted to adhesion and welding may be used as the balloon material. As compared with other plastic materials, the silicone is excellent in flexibility, expandability (an elongation percentage as much as 1000% is also attainable depending on the hardness), gas permeability, heat resistance, chemical resistance, and durability etc., and particularly, may provide excellent gas permeability so that an air removal work required earlier than an operation may be easily performed, permitting the balloon most suitable to the balloon catheter for medical treatments to be obtained.

### BRIEF DESCRIPTION OF THE DRAWINGS

FIG. 1 is an overall side view showing one embodiment of a non-profile balloon catheter according to the present invention, more specifically, a condition earlier than expansion of a balloon;
FIG. 2 is an overall side view showing a condition later than expansion of the balloon of the non-profile balloon catheter shown in FIG. 1:
FIG. 3 is a fragmentary enlarged side view of FIG. 1:
FIG. 4 is a fragmentary enlarged side view of FIG. 2:
FIG. 5 is a fragmentary sectional side view showing the non-profile balloon catheter according to the present invention, more specifically, one instance of a balloon catheter structured to be integral with a guide wire;
FIG. 6 is a fragmentary sectional side view showing one instance of a conventional balloon catheter structured to be integral with the guide wire;
FIG. 7 is an overall side view showing one conventional balloon catheter, more specifically, a condition earlier than expansion of the balloon;
FIG. 8 is an overall side view showing a condition later than expansion of the balloon of the balloon catheter shown in FIG. 7;
FIG. 9 is a fragmentary enlarged side view of FIG. 7: and
FIG. 10 is a fragmentary enlarged side view of FIG. 8.

### BEST MODE FOR CARRYING OUT THE INVENTION

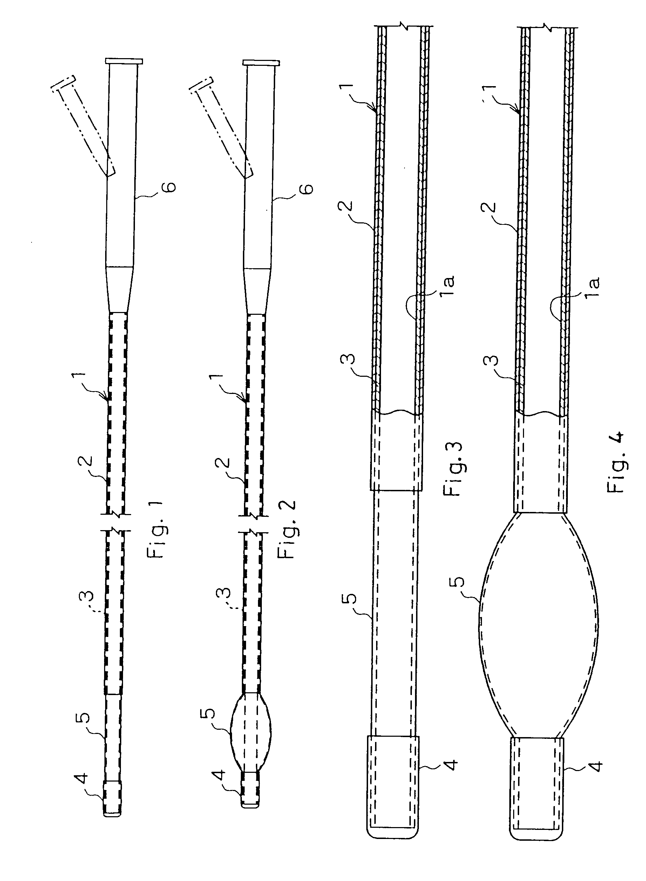

Hereinafter will be described the present invention based on one illustrated embodiment. FIGS. 1 to 4 are views showing a basic structure of a non-profile balloon catheter according to the present invention. FIG. 5 is a view specifically showing one of applications of the non-profile balloon catheter.

In the embodiment shown in FIGS. 1 to 4, the non-profile balloon catheter according to the present invention is composed of an outside hollow shaft (an outside tube) 2, an inside hollow shaft (an inside tube) 3, and a cap 4. The outside hollow shaft 2 and the inside hollow shaft 3 are combined to form a catheter shaft 1. The tip part of the inside hollow shaft 3 is projected forwards by a prescribed length from the tip end of the outside hollow shaft 2 to provide a projected portion, which is adapted to form a balloon 5. Further, the same connector 6 formed of a metal- or synthetic resin-made hard tube as the conventional connector is connected to the basal end of the shaft 1.

While a flexible and non-expansible material such as nylon and polyurethane may be used for the outside hollow shaft 2, like the conventional technology, it is preferably necessary to use PTFE (polytetrafluoroethylene) excellent in vital adaptability (anti-thrombus), surface smoothness, heat resistance, chemical resistance and durability etc.

While a flexible and expansible material such as nylon, polyethylene-polyolefin copolymer, polyethylene-terephtalate, and natural rubber may be used for the inside hollow shaft 3, like the conventional technology, it is preferably necessary to use silicone excellent in flexibility, expandability (an elongation percentage as much as 1000% is also attainable depending on the hardness), gas permeability, heat resistance, chemical resistance, and durability etc.

The cap 4 preferably requires use of a bottomed tube formed of the same material as the outside hollow shaft 2, and is adapted to prevent leakage of air or like fluid for balloon expandable actions by closing up an opening at the tip end of the inside hollow shaft 3 in such a manner that the cap 4 is secured with an adhesive etc., after being so put on as to cover the tip end of the inside hollow shaft 3. It is to be noted that the present invention is not limited to the above cap , and it is also allowable to close up the tip end of the inside hollow shaft 3 by adhesion and welding etc., after being crushed flat.

The double-tube catheter shaft 1 as described the above may be manufactured by extrusion molding, heat contraction, and immersion etc., thereby providing the double-tube catheter shaft 1 structured so that the tip part of the inside hollow shaft 3 is projected by a length corresponding to the balloon length from the tip end of the outside hollow shaft 2. Thus, the non-profile balloon catheter may be provided, in which the balloon 5 at the time when being in a non-expanded state is located at the inside away from the outside face of the shaft.

The catheter shaft 1 has therein a single lumen 1a formed of the inside hollow shaft 3, in which case, injecting the air or like fluid for balloon expandable actions into the lumen 1a through the connector 6 causes the projected portion at the tip end of the inside hollow shaft 3 to be expanded into the form of the balloon 5. When the supply of air etc. is stopped, the air etc. may be exhausted to the outside to cause the balloon 5 to be shrunken, permitting the balloon 5 to be housed into the outside hollow shaft 2. Further, a guide wire or medical instruments etc. are inserted into the lumen 1a through the connector 6, permitting various treatments to be performed.

FIG. 5 shows one instance of a balloon catheter structured to be integral with the guide wire, specifically, this type of balloon catheter needs to project its tip end from the tip end of the inside hollow shaft 3, with a guide wire 7 inserted into the inside hollow shaft 3, that is, the lumen 1a through the connector 6. The tip end of the guide wire 7 is fitted with a cap 8 that covers the guide wire 7 and the inside hollow shaft 3, and this cap 8 is secured with the adhesive etc. Thus, it is not possible to cause only the guide wire 7 to be pushed or pulled, resulting in need to hold the basal end side, that is, the user's hand side of the guide wire 7 for operating the catheter shaft 1. While insertion of the balloon catheter along the previously inserted guide wire normally takes place, use of the non-profile balloon catheter structured to be integral with the guide wire enables the balloon catheter to be inserted and placed by one attempt of inserting operation, permitting treatments etc. of high efficiency to be performed. The present invention is not limited to the guide wire, and it is also allowable to use other core materials.

Further, the balloon catheter of the present invention enables injection of the contrast medium and/or medicines etc. to the portion ahead of the catheter by providing one or more than two lumens (not shown) for the core material such as the guide wire 7, or alternatively, by providing the inside hollow shaft 3 in the form of the shaft having more than one lumen (not shown).

Furthermore, the present invention is not limited to the above guide wire, and it is also allowable to insert and place other medical instruments etc. in the inside hollow shaft 3.

Furthermore, soft metal such as Ni-Ti shape memory alloys and super-elastic alloys may be also used for the outside hollow shaft 2, permitting the non-profile catheter itself to be used as the guide wire.

It is a matter of course that the non-profile balloon catheter of the present invention is not limited to the illustrated embodiments described the above, and it is also allowable to adopt other various modes in the present invention.

## Claims

1. A balloon catheter having an expandable balloon at the tip part of a shaft, **characterized in that** a hollow shaft formed of a balloon material is inserted into a hollow shaft formed of a shaft material, and the tip part of the inside hollow shaft is projected from the tip end of the outside hollow shaft so that the expandable balloon is formed of the projected portion of the inside hollow shaft.

2. The balloon catheter according to claim 1, wherein the tip end of the inside hollow shaft is closed up with a cap formed of the shaft material.

3. The balloon catheter according to claim 1 or 2, wherein after insertion of a guide wire or other core material into the inside hollow shaft, said guide wire or other core material is secured to the tip part of the inside hollow shaft.

4. The balloon catheter according to claim 3, wherein the guide wire or other core material is provided with one or more lumens.

5. The balloon catheter according to any one of claims 1 to 3, wherein the inside hollow shaft is provided with more than one lumen.

6. The balloon catheter according to any one of claims 1 to 5, wherein the outside hollow shaft is formed of polytetrafluoroetylene.

7. The balloon catheter according to any one of claims 1 to 6, wherein the inside hollow shaft is formed of silicone.

8. The balloon catheter according to any one of claims 1 to 5 and 7, wherein the outside hollow shaft is formed of soft metal.
